# EUROPEAN PATENT SPECIFICATION

(11) **EP 1 267 908 B1**
(45) Date of publication and mention of the grant of the patent: **06.05.2004**
(21) Application number: 01919363.0
(22) Date of filing: 01.03.2001
(51) Int. Cl.: A61K 39/00, A61P 9/10

(54) **VACCINE FOR THE TREATMENT OF ATHEROSCLEROSIS**
IMPSTOFF ZUR BEHANDLUNG VON ATHEROSCLEROSIS
VACCIN POUR LE TRAITEMENT DE L'ATHEROSCLEROSE

(30) Priority: 03.03.2000 GB 0005240; 07.09.2000 GB 0022005
(43) Date of publication of application: 02.01.2003
(73) Proprietor: GlaxoSmithKline Biologicals S.A., 1330 Rixensart (BE)
(72) Inventor: FRUCHART, Jean-Charles, Institut Pasteur de Lille, F-59019 Lil le Cedex (FR); MONTEYNE, Philippe, GlaxoSmithKline, B-1330 Rixensart (BE); PALMANTIER, Remi, GlaxoSmithKline, B-1330 Rixensart (BE); VAN MECHELEN, Marcelle, Paulette, GlaxoSmithKline, B-1330 Rixensart (BE)
(74) Representative: Lubienski, Michael John
(86) International application number: PCT/EP2001/002326
(87) International publication number: WO 2001/064008

(56) References cited:
- WO-A-87/02062
- WO-A-93/00443
- WO-A-98/07751
- WO-A-99/36785
- WO-A-99/50660
- WO-A-99/67637
- US-A- 5 424 068
- DAVIGNON JEAN: "Advances in drug treatment of dyslipidemia: Focus on atorvastatin." CANADIAN JOURNAL OF CARDIOLOGY, vol. 14, no. SUPPL. B, May 1998 (1998-05), pages 28B-38B, XP001029508 ISSN: 0828-282X

## Description

The present invention relates to novel vaccine therapies, and prophylactic treatments of atherosclerotic diseases. Accordingly there is provided, immunogens comprising apolipoproteins, or fragments or derivatives thereof, which in their native form the whole apolipoprotein has at least one of the following activities: (a) the inhibition of the binding of Apolipoprotein B to its receptor, and, or, (b) the inhibition of lipoprotein lipase. A preferred example of one such apolipoprotein is Apolipoprotein C-III (ApoCIII). The vaccines of the present invention, comprising said immunogens, are potent in the prevention, or reduction, of atherosclerotic plaque formation over prolonged periods of time, thereby reducing the potential of atheroslerosis leading to coronary or cerebrovascular disease. Accordingly, there is provided a novel method of treating atherosclerosis by selectively inhibiting the activity of specific apolipoproteins in a human host, and in a preferred aspect of the invention the apolipoprotein is ApoCIII, comprising administering an agent which results in the selective inhibition of the apolipoprotein activity to a human. Also provided are methods of treating or preventing atherosclerosis by active vaccination, or passive vaccination through administration to a patient of an antibody that is capable of binding to an apolipoprotein that has at least one of the following activities: (a) the inhibition of the binding of Apolipoprotein B to its receptor, and, or, (b) the inhibition of lipoprotein lipase; and in this aspect of the present invention the antibody preferably binds to and abrogates the activity of ApoCIII. There is further provided the use of the immunogens or vaccines of the present invention in medicine, and methods of their production.

Atherosclerosis is the leading cause of death and disability in the developed world, and is the major cause of coronary and cerebrovascular deaths, with approximately 7.2 and 4.6 million deaths per year worldwide respectively (Atherosclerosis is generally described in Harrison's Principles of Internal Medicine (14^{th} Edition, McGraw Hill, p1345-1352), Berliner, J. et al., 1995, Circulation, 91:2488-2496; Ross, R., 1993; Nature, 362:801). The name in Greek refers to the thickening (sclerosis) of the arterial intima and accumulation of lipid (athere) in lesions.

Although many generalised or systemic risk factors predispose to its development, such as a high cholesterol diet and smoking, this disease may affect different distinct regions of the circulation. For example, atherosclerosis of the coronary arteries commonly causes angina pectoris and myocardial infarction. Whilst, atherosclerosis of the arteries supplying the central nervous system frequently provokes transient cerebral ischemia and strokes. In the peripheral circulation, atherosclerosis can cause intermittent claudication and gangrene and can jeopardise limb viability. Involvement of the splanchnic circulation can cause mesenteric ischemia and bowel infarction. Atherosclerosis can affect the kidney directly (eg causing renal artery stenosis), and in addition, the kidney is a frequent site of atheroembolic disease.

Atherogenesis in humans typically occurs over many years, usually many decades. The slow build up of atherogenic plaques in the lining of the vasculature can lead to chronic clinical expressions through blood flow restriction (such as stable effort-induced angina pectoris or predictable and reproducible intermittent claudication). Alternatively, a much more dramatic acute clinical event, such as a myocardial infarction or cerebrovascular accident can occur after plaque rupture. The way in which atherosclerosis affects an arterial segment also varies, an additional feature of the heterogeneity and complexity of this disease. Atheromas are usually thought of as stenotic lesions, or plaques, which can limit blood flow, however, atherosclerosis can also cause ectasia and development of aneurysmal disease with an increase in lumen caliber. This expression of atherosclerosis frequently occurs in the aorta, creating a predisposition to rupture or dissection rather than to stenosis or occlusion.

The genesis of atherogenic plaques has been studied in depth. In normal human adults, the intimal layer of arteries contains some resident smooth muscle cells embedded in extracellular matrix and is covered with a monolayer of vascular endothelial cells. Initial stages of atherogenesis involve the development of "fatty streaks" in the walls of the blood vessel resulting from accumulation and deposit of lipoproteins in regions of the intimal layer of the artery. Low-density lipoprotein (LDL) particles, rich in cholesterol, is an example of an atherogenic lipoprotein which is capable of deposition in the vessel walls to form such fatty streaks.

Once deposited within the vessel wall, the lipoprotein particles undergo chemical modification, including both oxidation and non-enzymatic glycation. These oxidised and glycated lipoproteins then contribute to many of the subsequent events of lesion development. The chemical modifications attract macrophages within the vessel walls, which internalise the oxidised LDL and become foam cells which initiate lesions called plaques. It is the atherosclerotic plaques which are responsible for the clinical manifestations of atherosclerosis, either they limit blood flow, or allow aneurism, or may even rupture provoking the coronary or cerebrovascular attacks.

The development of atherosclerosis is a long process which may occur over decades, which is initiated by an imbalance between atherogenic and protective lipoproteins. For example, cholesterol associated with high-density lipoproteins or HDL (so called "good" cholesterol) and low-density lipoproteins or LDL (so called "bad" cholesterol) levels in the circulation are thought to be markers of increased probability of atherosclerosis (Harrison's Principles of Internal Medicine (14^{th} Edition, McGraw Hill, p 1345-1352)).

Cholesterol, cholesterol esters, triacylglycerols and other lipids are transported in body fluids by a series of lipoproteins classified according to their increasing density: chylomicrons, Very Low, Low, Intermediate and High density lipoproteins (CM, VLDL, LDL, IDL and HDL respectively). These lipoprotein-complexes consist of a core of hydrophobic lipids surrounded by polar lipids and then by a shell of Apolipoproteins. Currently, there are ten types of apolipoproteins known, A-I, A-II, B, CI, CII, CIII, D, E, H and J. There are at least two functions of these apolipoproteins which are common to all lipoprotein complexes, first they are responsible for the solubilisation of the hydrophobic lipid cores that they carry, and second they are also involved in the regulation of cholesterol lipoprotein uptake by specific cells. The different different types of lipoproteins may have different functions, for example LDL are rich in cholesterol esters are thought to be associated with the transport of cholesterol to peripheral tissues for new membrane synthesis.

One of these apolipoproteins, apolipoprotein C-III (ApoCIII), is a 79 amino acid protein produced in the liver and intestine (Brewer *et al*., J. Biol. Chem. (1974), 249 : 4975-4984; Protter, A.A., et al., 1984, DNA, 3:449-456; Fruchart, J.C. et al, 1996, Drugs Affecting Lipid Metabolism, (Eds. Gotto, A.M. et al.), Kluwer Academic Publishers and Fordazione Giovanni Lorenzini, Netherlands, p631-638; Claveny, V. et al., Arteriosclerosis, Thrombosis and Vascular Biology, 15, 7, 963-971; US patent No. 4,801,531; McConathy, W.J. et al. 1992, Journal of Lipid Research, 33, 995-1003). Apo CIII is a component of CM, VLDV and LDL (Lenich *et al*., C., J. Lip. Res. (1988) 29, 755-764) which exists as three isoforms : apo CIII0, apo CIII1 and apo CIII2. Apo CIII is not glycosylated, however apo CIII1 and apo CIII2 are glycosylated and have respectively one and two sialic acid residues (Ito *et al*., 1989 J.lipd. Res. Nov 30:11 1781-1787). The sugar moiety consists of disaccharide β-D galactosyl (1-3) α-N-Acetyl-D-Galactosamine attached to threonine 74 of protein chain by O-glycosidic binding (Assman *et al*., 1989, BBA 541:234-240). In human normolipidemic plasma apo CIII0, apo CIII1 and apo CIII2 represent 14%, 59% and 27% of total apo CIII respectively. Mutagenesis of the glycosylation site of human apo CIII doesn't affect its secretion and lipid binding (Roghani et al., 1988 JBC 34:17925-32).

Human ApoCIII has the following amino acid sequence: SEAEDASLLSFMQGYMKHATKTAKDALSSVQESQVAQQARGWVTDGFSSLK DYWSTVKDKFSEFWDLDPEVRPTSAVAA (SEQ ID.NO. 1) Plasma concentration of apo CIII is positively correlated with levels of plasma triglycerides (Schonfeld *et al*., Metabolism (1979) 28 : 1001-1010; Kaslyap *et al*., J. Lip. Res. (1981) 22 : 800-810). Liver perfusion studies demonstrate that apo CIII inhibits the hepatic uptake of triglyceride-rich lipoproteins (TRL) and their remnants (Shelburne *et al*., J. Clin. Inves., (1980) 65 : 652-658, Windler *et al*., J. Lip. Res. (1985) 26 : 556-563). Also *in vitro* experiments show that apo CIII inhibit the activity of both lipoprotein lipase (LPL) and hepatic lipase (Brown and Bakinsky, Biochim. Biophs. Acta. (1972) 46 : 375-382; Krauss *et al.,* Circ. Res. (1973) 33 : 403-411; Wang *et al*., J. Clin. Inves. (1985) 75 : 384-390; Mc Conathy *et al*., J. Lip. Res. (1972) 33 : 995-1003; Kinnemen and Enholm, FEBS (1976) 65 : 354-357). Moreover, ApoCIII is said to be involved in inhibition of LDL binding to LDL receptors (Fruchart et al. *supra*), via ApoB.

The role of apo CIII in plasma TRL metabolism has been more defined by the results of recent studies in transgenic animals (Aalto-Setälä *et al*., J. Clin. Invest. (1992) 90:5 1889-1900.). Plasma accumulation of TRL in mice overexpressing apo CIII has been shown to be associated with reduced plasma VLDL and chylomicron clearance (Harrold *et al*., J. Lip. Res. (1996) 37 : 754-760) also the inhibitory effect of C apolipoproteins on the LDL receptor of apo B-containing lipoproteins was demonstrated (Clavey *et al*., Arth. Thromb. and Vasc. Biol. (1995) 15 : 963-971).

Previous vaccines in the field of immunotherapy of atherosclerosis have focused on the use of cholesterol as an immunogen to reduce serum cholesterol levels (Bailey, J.M. *et al*., 1994, Biochemical Society Transactions, 22, 433S; Alving, C. and Swartz, G.M., 1991, Crit. Rev. Immunol., 10, 441-453; Alving, C. and Wassef, N.M., 1999, Immunology Today, 20, 8, 362-366). Others have attempted to alter the activity of the Cholesterol Ester Transfer Protein (CETP) by vaccination (WO 99/15655). Alternatively, some authors have described vaccines using oxidised LDL as the immunogen, in order to inhibit plaque formation after balloon injury in hypercholesterolemic rabbits (Nilsson, J. *et al*., 1997, JACC, 30, 7, 1886-1891).

It has been found, surprisingly, that atherosclerosis may be prevented or ameliorated by active or passive immunotherapy, by reducing or blocking the function of certain atherosclerosis promoting apolipoproteins.

The present invention provides immunogens effective in the prophylaxis or therapy of atherosclerosis, and also provides for methods of treatment of atherosclerosis by the administration of the immunogens of the present invention to individuals in need thereof.

The immunogens of the present invention comprise apolipoproteins selected from those apolipoproteins which promote the formation of atherosclerotic plaques in individuals suffering from or disposed towards atherosclerosis. The apolipoproteins which are selected to form the basis of preferred immunogens of the present invention are apolipoproteins which have at least one of the following two properties: the native apolipoprotein is capable of (a) inhibiting the binding of Apolipoprotein B to its receptor, and/or (b) the inhibition of the enzyme lipoprotein lipase.

The apolipoproteins which may be used as immunogens of the present invention may have either one of the above activities, but most preferably have both activities. The most preferred immunogen which has both activities comprises Apolipoprotein CIII (ApoCIII).

The activity of any given apolipoprotein in the inhibition of apo B containing lipoproteins binding to its receptor, or in the enzymatic activity of lipoprotein lipase, may be investigated using techniques that are known in the art. Examples of these techniques are described in Fruchart *et al*, *supra*; McConathy *et al., supra*; Shelburne *et*. *al*. *supra* and Windler *et. al*. *supra*.

The immunogens of the present invention may comprise the whole length native apolipoprotein, or may alternatively comprise fragments, peptides or mimotopes thereof, which retain the functional activity of the therapy or prophylaxis of atherosclerosis.

For example, the immunogen may be full length, or may comprise fragments of the native apolipoprotein which are shorter than the whole length of the native apolipoprotein. Preferably the fragments of the whole length proteins are less than 80 amino acids in length, more preferably less than 50 amino acids, more preferably less than 40 amino acids and most preferably within the range of 4 to 25 amino acids long.

The apolipoprotein fragments which may be used as immunogens of the present invention share the function of the whole length apolipoprotein, of being able (when suitably presented to the immune system) to induce anti-Apolipoprotein antibodies. Moreover, the antibodies induced by the fragments are functional in the treatment of atherosclerosis, and in a preferred form of the present invention they abrogate the inhibition exerted by the apolipoprotein on the binding of ApoB to its receptor, and/or the activity of lipoprotein lipase.

Peptides, which may be formulated into immunogens of the present invention may be isolated from surface exposed epitopes of the apolipoproteins. The present inventors have found that the peptides useful in the present invention, are also found to be highly surface exposed epitopes. From this observation the present inventors have designed a method for providing other suitable epitopes, those being epitopes having highly accessible regions calculated over a sliding window of five residues. The inventors have found that preferred regions of the apolipoproteins have an accessible surface calculated over a sliding window of 5 residues using the Molecular Simulations Software (MSI) of greater than 50 Å², and preferably greater than 80Å².

Peptides incorporating the amino acid sequence of such surface exposed epitopes form an aspect of the present invention. Mimotopes which have the same characteristics as these peptides, and immunogens comprising such mimotopes which generate an immune response which cross-react with the apolipoprotein, also form part of the present invention.

The immunogens of the present invention may, therefore, comprise the isolated peptides encompassing the apolipoprotein epitopes themselves, and any mimotope thereof. The meaning of mimotope is defined as an entity which is sufficiently similar to the apolipoprotein epitope so as to be capable of being recognised by antibodies which recognise the apolipoprotein; (Gheysen, H.M., et al., 1986, Synthetic peptides as antigens. Wiley, Chichester, Ciba foundation symposium 119, p130-149; Gheysen, H.M., 1986, Molecular Immunology, 23,7, 709-715); or are capable of raising antibodies, when coupled to a suitable carrier, which antibodies cross-react with the native apolipoprotein.

Peptide mimotopes of the above-identified apolipoprotein epitopes may be designed for a particular purpose by addition, deletion or substitution of elected amino acids. Thus, the peptides of the present invention may be modified for the purposes of ease of conjugation to a protein carrier. For example, it may be desirable for some chemical conjugation methods to include a terminal cysteine to the apolipoprotein epitope. In addition it may be desirable for peptides conjugated to a protein carrier to include a hydrophobic terminus distal from the conjugated terminus of the peptide, such that the free unconjugated end of the peptide remains associated with the surface of the carrier protein. This reduces the conformational degrees of freedom of the peptide. and thus increases the probability that the peptide is presented in a conformation which most closely resembles that of the apolipoprotein peptide as found in the context of the whole apolipoprotein. For example, the peptides may be altered to have an N-terminal cysteine and a C-terminal hydrophobic amidated tail. Alternatively, the addition or substitution of a D-stereoisomer form of one or more of the amino acids may be performed to create a beneficial derivative, for example to enhance stability of the peptide. Those skilled in the art will realise that such modified peptides, or mimotopes, could be a wholly or partly non-peptide mimotope wherein the constituent residues arc not necessarily confined to the 20 naturally occurring amino acids. in addition, these may be cyclised by techniques known in the art to constrain the peptide into a conformation that closely resembles its shape when the peptide sequence is in the context of the whole apolipoprotein.

The peptide mimotopes may also be retro sequences of the natural apolipoprotein peptide sequences, in that the sequence orientation is reversed; or alternatively the sequences may be entirely or at least in part comprised of D-stereo isomer amino acids (inverso sequences). Also, the peptide sequences may be retro-inverso in character, in that the sequence orientation is reversed and the amino acids are of the D-stereoisomer form. Such retro or retro-inverso peptides have the advantage of being non-self, and as such may overcome problems of self-tolerance in the immune system.

Alternatively, peptide mimotopes may be identified using antibodies which are capable themselves of binding to the apolipoprotein, using techniques such as phage display technology (EP 0 552 267 B1). This technique, generates a large number of peptide sequences which mimic the structure of the native peptides and are, therefore, capable of binding to anti-native peptide antibodies, but may not necessarily themselves share significant sequence homology to the native apolipoprotein.

The most preferred immunogens of the present invention comprise ApoCIII or fragment, peptide or mimotope thereof.

The ApoCIII the immunogen may be the full length native protein: Human ApoCIII 1-79,

Alternatively, the immunogen may comprise fragments of the whole ApoCIII which are 78 amino acids long or less, preferably 50 amino acids long or less, more preferably 40 amino acids or less, and most preferably within the range of 4 to 25 amino acids long. Particularly preferred fragments or peptides will include the region defined by amino acids 1-17, 1-40, 12-35, 41-79, 45-65, or 45-76 in the mature ApoCIII. The peptide sequences for some of the preferred peptides are:

An immunologically equivalent fragment of ApoCIII may be defined as a smaller polypeptide than ApoCIII itself, which is capable of generating immune responses that recognise native ApoCIII, and which function in the therapy or prophylaxis of atherosclerosis.

Other apolipoproteins which may be used in the immunogens of the present invention are Apolipoprotein CII or Apolipoprotein E.

In one particularly preferred embodiment of the present invention the apolipoprotein or fragment thereof is linked to a carrier molecule to enhance the immunogenicity of the apolipoprotein or fragment thereof. Accordingly, the peptides or mimotopes may be linked via chemical covalent conjugation or by expression of genetically engineered fusion partners, optionally via a linker sequence. The peptides may have two or more Glycine residues as a linker sequence, and often have a terminal exposed cystein residue for linkage purposes. For example, some of these preferred peptides are listed below:

The covalent coupling of the apolipoprotein, such as ApoCIII, to the carrier protein can be carried out in a manner well known in the art. Thus, for example, for direct covalent coupling it is possible to utilise a carbodiimide, glutaraldehyde or (N-[γ-maleimidobutyryloxy]) succinimide ester, utilising common commercially available heterobifunctional linkers such as CDAP and SPDP (using manufacturers instructions). After the coupling reaction, the immunogen can easily be isolated and purified by means of a dialysis method, a gel filtration method, a fractionation method etc.

The types of carriers used in the immunogens of the present invention will be readily known to the man skilled in the art. The function of the carrier is to provide cytokine help in order to enhance the immune response against the apolipoprotein or apolipoprotein peptide. A non-exhaustive list of carriers which may be used in the present invention include: Keyhole limpet Haemocyanin (KLH), serum albumins such as bovine serum albumin (BSA), inactivated bacterial toxins such as tetanus or diptheria toxins (TT and DT), or recombinant fragments thereof (for example, Domain 1 of Fragment C of TT, or the translocation domain of DT), or the purified protein derivative of tuberculin (PPD). Alternatively the apolipoprotein or mimotopes or epitopes may be linked to the carrier in a non-covalent fashion such as association via a liposome carrier or by co-adsorbtion onto an aluminium salt, which may additionally comprise immunogens capable of providing T-cell help or additional adjuvant immunostimulators. Preferably the ratio of the number of apolipoprotein, or fragment or peptide thereof, to carrier protein is in the order of 1:1 to 20:1, and preferably each carrier should carry between 3-15 apolipoproteins, or peptide or fragment thereof.

In an embodiment of the invention the carrier is Protein D from Haemophilus influenzae (EP 0 594 610 B1). Protein D is an IgD-binding protein from Haemophilus influenzae and has been patented by Forsgren (WO 91/18926, granted EP 0 594 610 B1). In some circumstances, for example in recombinant immunogen expression systems it may be desirable to use fragments of protein D, for example Protein D 1/3^{rd} (comprising the N-terminal 100-110 amino acids of protein D (WO 99/10375; WO 00/50077)).

Another preferred method of presenting apolipoprotein, such as ApoCIII, or the peptides of the present invention, is in the context of a recombinant fusion molecule. For example, EP 0 421 635 B describes the use of chimeric hepadnavirus core antigen particles to present foreign peptide sequences in a virus-like particle. As such, immunogens of the present invention may comprise apolipoprotein or apolipoprotein peptides presented in chimeric particles consisting of hepatitis B core (HepB core) antigen. Additionally, the recombinant fusion proteins may comprise the mimotopes of the present invention and a carrier protein, such as NS 1 of the influenza virus. For any recombinantly expressed protein which forms part of the present invention, the nucleic acid which encodes said immunogen also forms an aspect of the present invention.

Accordingly, preferred immunogens of the present invention comprise the peptide SEQ ID NO. 1 or SEQ ID NO. 2-7, presented in a recombinant expression system (such as HepB core) or conjugated to a carrier protein, such that the recombinant expression system or the carrier protein provide T-cell help for generation of an immune response to SEQ ID NO. 1 or SEQ ID NO. 2-7. Particularly preferred immunogens comprise SEQ ID NO. 4 alone, or conjugated or fused to a carrier protein to provide T-cell help for generation of an immune response to SEQ ID NO. 4.

In an alternative embodiment of the present invention the immunogenicity of the peptides is enhanced by the addition of T-helper (Th) epitopes. The immunogens of the present invention may, therefore, comprise the peptides as described previously and promiscuous Th epitopes either as chemical conjugates or as purely synthetic peptide constructs. The apolipoprotein peptides are preferably joined to the Th epitopes via a spacer (e.g., Gly-Gly) at either the N- or C-terminus of the apolipoprotein peptide. The immunogens may comprise 1 or more promiscuous Th epitopes, and more preferably between 2 to 5 Th epitopes.
A Th epitope is a sequence of amino acids that comprise a Th epitope. A Th epitope can consist of a continuous ir discontinuous epitope. Hence not every amino acid of Th is necessarily part of the epitope. Th-epitopes that are promiscuous are highly and broadly reactive in animal and human populations with widely divergent MHC types (Partidos et al. (1991) "Immune Responses in Mice Following Immunization with Chimeric Synthetic Peptides Representing B and T Cell Epitopes of Measles Virus Proteins" J. of Gen. Virol. 72:1293-1299

US 5,759,551). The Th domains that may be used in accordance with the present invention have from about 10 to about 50 amino acids, and preferably from about 10 to about 30 amino acids. When multiple Th epitopes are present, each Th epitope is independently the same or different.

Th epitopes include as examples, pathogen derived epitopes such as Hepatitis surface or core (peptide 50-69, Ferrari *et al*., J.Clin.Invest, 1991, 88, 214-222) antigen Th epitopes, Pertussis toxin Th epitopes, tetanus toxin Th epitopes (such as P2 (EP 0 378 881 B1) and P30 (WO 96/34888, WO 95/31480, WO 95/26365), measles virus F protein Th epitopes, Chlamidia trachomatis major outer membrane protein Th epitopes (such as P11, Stagg et al., Immunology, 1993, 79, 1-9), Yersinia invasin and diptheria toxin Th epitopes. Other Th epitopes are described in US 5,759,551 and Cease et al., 1987, Proc. Natl. Acad. Sci. 84, 4249-4253; and Partidos et al., J.Gen.Virol, 1991, 72, 1293-1299; WO 95/26365 and EP 0 752 886 B.

Peptides used in the present invention can be readily synthesised by solid phase procedures well known in the art. Suitable syntheses may be performed by utilising "T-boc" or "F-moc" procedures. Cyclic peptides can be synthesised by the solid phase procedure employing the well-known "F-moc" procedure and polyamide resin in the fully automated apparatus. Alternatively, those skilled in the art will know the necessary laboratory procedures to perform the process manually. Techniques and procedures for solid phase synthesis are described in 'Solid Phase Peptide Synthesis: A Practical Approach' by E. Atherton and R.C. Sheppard, published by IRL at Oxford University Press (1989). Alternatively, the peptides may be produced by recombinant methods, including expressing nucleic acid molecules encoding the mimotopes in a bacterial or mammalian cell line, followed by purification of the expressed mimotope. Techniques for recombinant expression of peptides and proteins are known in the art, and are described in Maniatis, T., Fritsch, E.F. and Sambrook et al., Molecular cloning, a laboratory manual, 2nd Ed.; Cold Spring Harbor Laboratory Press, Cold Spring Harbor, New York (1989).

Also forming part of the present invention are portions of nucleic acid which encode the immunogens of the present invention or peptides, mimotopes or derivatives thereof, or recombinant fusion proteins comprising the immunogens. In particular isolated nucleic acid molecules which encode SEQ ID. NO. 1-7, or immunogens comprising SEQ ID NOs. 1-7 are provided.

The immunogens of the present invention are provided for use in medicine, for use in the treatment of atherosclerosis, and for formulation into immunogenic compositions or vaccines of the present invention.

The immunogens of the present invention may be formulated into immunogenic compositions or vaccines, which are effective in the prophylaxis or therapy of atherosclerosis. The immunogenic compositions and vaccines comprise one or more immunogens of the present invention as previously described. Accordingly the vaccines comprise apolipoproteins selected from those apolipoproteins which promote the formation of artherosclerotic plaques. Preferred vaccines comprise immunogens comprising apolipoproteins, which have at least one of the following two properties: the native apolipoprotein is active in the suppression of ApoB binding to its receptor, and/or is capable of inhibiting the enzymatic activity of lipoprotein lipase.

The most preferred vaccines or immunogenic compositions of the present invention comprise ApoCIII, or fragment or peptide thereof. Most preferably, the vaccine comprises ApoCIII or fragment thereof, conjugated or fused to a carrier protein to provide T-cell help for generation of an immune response against the ApoCIII or fragment thereof.

Vaccines or immunogenic compositions of the present invention, may advantageously also include an adjuvant. Suitable adjuvants for vaccines of the present invention comprise those adjuvants that are capable of enhancing the antibody responses against the apolipoprotein immunogen. Adjuvants are well known in the art (Vaccine Design - The Subunit and Adjuvant Approach, 1995, Pharmaceutical Biotechnology, Volume 6, Eds. Powell, M.F., and Newman, M.J., Plenum Press, New York and London, ISBN 0-306-44867-X). Preferred adjuvants for use with immunogens of the present invention include aluminium or calcium salts (for example hydroxide or phosphate salts). Preferred adjuvants for use with immunogens of the present invention include: aluminium or calcium salts (hydroxide or phosphate), oil in water emulsions (WO 95/17210, EP 0 399 843), or particulate carriers such as liposomes (WO 96/33739). Immunologically active saponin fractions (e.g. Quit A) having adjuvant activity derived from the bark of the South American tree Quillaja Saponaria Molina are particularly preferred. Derivatives of Quil A, for example QS21 (an HPLC purified fraction derivative of Quil A), and the method of its production is disclosed in US Patent No.5,057,540. Amongst QS21 (known as QA21) other fractions such as QA17 are also disclosed. 3 De-O-acylated monophosphoryl lipid A (3D-MPL) is a well known adjuvant manufactured by Ribi Immunochem, Montana. It can be prepared by the methods taught in GB 2122204B. A preferred form of 3D-MPL is in the form of an emulsion wherein the 3D-MPL has a small particle size of less than 0.2µm in diameter (EP 0 689 454 B1).

Adjuvants also include, but are not limited to, muramyl dipeptide and saponins such as Quil A, bacterial lipopolysaccharides such as 3D-MPL (3-O-deacylated monophosphoryl lipid A), or TDM. As a further exemplary alternative, the protein can be encapsulated within microparticles such as liposomes, or in non-particulate suspensions of polyoxyethylene ether (WO 99/52549). Particularly preferred adjuvants are combinations of 3D-MPL and QS21 (EP 0 671 948 B1), oil in water emulsions comprising 3D-MPL and QS21 (WO 95/17210, PCT/EP98/05714), 3D-MPL formulated with other carriers (EP 0 689 454 B1), or QS21 formulated in cholesterol containing liposomes (WO 96/33739), or immunostimulatory oligonucleotides (WO 96/02555).

The vaccines of the present invention will be generally administered for both priming and boosting doses. It is expected that the boosting doses will be adequately spaced, or preferably given yearly or at such times where the levels of circulating antibody fall below a desired level. Boosting doses may consist of the peptide in the absence of the original carrier molecule. Such booster constructs may comprise an alternative carrier or may be in the absence of any carrier.

In a further aspect of the present invention there is provided a vaccine or immunogenic composition as herein described for use in medicine.

The immunogenic composition or vaccine preparations of the present invention may be used to protect or treat a mammal susceptible to, or suffering from atherosclerosis, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts.

The amount of protein in each vaccine or immunogenic composition dose is selected as an amount which induces an immunoprotective response without significant, adverse side effects in typical vaccinees. Such amount will vary depending upon which specific immunogen is employed and how it is presented. Generally, it is expected that each dose will comprise 1-1000 µg of protein, preferably 1-500 µg, preferably 1-100 µg, of which 1 to 50µg is the most preferable range. An optimal amount for a particular vaccine can be ascertained by standard studies involving observation of appropriate immune responses in subjects. Following an initial vaccination, subjects may receive one or several booster immunisations adequately spaced.

Vaccine preparation is generally described in New Trends and Developments in Vaccines, edited by Voller et al., University Park Press, Baltimore, Maryland, U.S.A. 1978. Conjugation of proteins to macromolecules is disclosed by Likhite, U.S. Patent 4,372,945 and by Armor et al., U.S. Patent 4, 474,757.

Ligands which are capable of binding to the same apolipoproteins which form the basis for the immunogens of the present invention form an important embodiment part of the present invention. Such ligands are capable of being used in passive prophylaxis or therapy, by administration of the ligands into a patient, for the amelioration of atherogenic disease.

Accordingly, the preferred ligands of the present invention are capable of binding to, and limiting the activity of, apolipoproteins which have at least one of the following two properties: the native apolipoprotein is capable of suppressing the binding of Apolipoprotein B to its receptor, and/or is capable of inhibiting the enzymatic activity of lipoprotein lipase.

The ligands of the present invention preferably bind to apolipoproteins which may have either one of the above activities, but most preferably have both activities. A particularly preferred ligand is one that binds to Apolipoprotein CIII (ApoCIII).

Preferred examples of such useful ligands include monoclonal or polyclonal antibodies. For example, antibodies induced in one animal may be purified and passively administered to another animal for the prophylaxis or therapy of atherosclerosis. The immunogens of the present invention may also be used for the generation of monoclonal antibody hybridomas (using known techniques e.g. Köhler and Milstein, Nature, 1975, 256, p495), humanised monoclonal antibodies or CDR grafted monoclonals, by techniques known in the art.

The term "antibody" herein is used to refer to a molecule having a useful antigen binding specificity. Those skilled in the art will readily appreciate that this term may also cover polypeptides which are fragments of or derivatives of antibodies yet which can show the same or a closely similar functionality. Such antibody fragments or derivatives are intended to be encompassed by the term antibody as used herein.

Accordingly there is provided by the present invention, an isolated antibody generated against the isolated immunogens of the present invention.

There is also provided by the present invention a poly or monoclonal antibody preparation, that binds to ApoCIII. Particularly preferred poly or monoclonal antibodies recognise fragments of the whole native ApoCIII, such as Human ApoCIII Hybrodomas secreting the monoclonal antibody ligands of the present invention are also provided.

Pharmaceutical compositions comprising the ligands, described above, also form an aspect of the present invention. Also provided are the use of the ligands in medicine, and in the manufacture of medicaments for the treatment of atherosclerosis.

In the passive treatments of atherosclerosis as provided herein, the administration of the ligands or antibodies of the present invention will be administered intra-venously to the patients in need thereof. The frequency of administration may be determined clinically by following the decline of antibody titres in the serum of patients over time, but in any event may be at a frequency of 1 to 52 times per year, and most preferably between 1 and 12 times per year. Quantities of antibody or ligand may vary according to the severity of the disease, or half-life of the antibody in the serum, but preferably will be in the range of 1 to 10 mg/kg of patient, and preferably within the range of 1 to 5 mg/kg of patient, and most preferably 1 to 2 mg/kg of patient.

The immunogens, immunogenic compositions, vaccines or ligands of the present invention may be administered to a patient who is suffering from, or is at risk to, atherosclerotic disease, and are effective in re-establishing the correct equilibrium of the "bad" lipoproteins (apo B containing lipoproteins) to the "good " lipoproteins (apo A-1 containing lipoproteins) balance, and minimise the circulation time of apo B containing lipoproteins. Not wishing to be bound by theory, the inventors believe that these functions minimise the possibility of deposit and oxidation of apo B containing lipoproteins within the blood vessel walls, and hence, reduce the risk of atherosclerotic plaque formation or growth.

The present invention, therefore, provides the use of the apolipoprotein immunogens of the present invention (as defined above), in the manufacture of pharmaceutical compositions for the prophylaxis or therapy of atherosclerosis. Immunogens comprising apolipoprotein or peptides of the present invention, and carrier molecules are also provided for use in the manufacture of vaccines for the immunoprophylaxis or therapy of atherosclerosis. Accordingly, the apolipoprotein immunogens of the present invention are provided for use in medicine, and in the medical treatment or prophylaxis of atherosclerosis.

There is also provided a method of treatment or prophylaxis of atherosclerosis comprising the administration to a patient suffering from or susceptible to atherosclerosis, of an immunogenic composition or vaccine or ligand of the present invention.

A method of prophylaxis or treatment of atherosclerosis is provided which comprises a reduction of total circulating triglyceride levels in a patient, by the administration of a vaccine of the present invention to the patient. In particular there is provided a method of reducing the amount of circulating VLDL and LDL in a patient, by the administration of the vaccine or ligands of the present invention to the patient.

Also provided is a method of prophylaxis or treatment of atherosclerosis by the administration to a patient of a vaccine which is capable of reducing the average circulation time of ApoB containing lipoproteins. In this regard the average circulation time of ApoB containing lipoproteins, may be investigated in an *in vivo* animal model by the measuring the clearance rate of labelled ApoB containing lipoproteins from the plasma of the mammal (half-life of labelled ApoB containing lipoproteins).

A preferred immunogen for these method of treatment aspects of the present invention comprises ApoCIII. Surprisingly, the targetting of ApoCIII by the vaccine or the ligand downregulates the negative effects of the "bad" cholesterol (LDL), whilst not having a negative effect on the "good" cholesterol (HDL).

There is provided by the present invention a method of treatment or prophylaxis of atherosclerosis by selectively inhibiting the activity of an atherogenic apolipoprotein in a human host, comprising administering an agent which results in the inhibition of at least one of the following activities of said atherogenic apolipoprotein in the human host in need of such treatment or prophylaxis, (a) the inhibition of the binding of Apolipoprotein B to its receptor, and, or (b) the inhibition of lipoprotein lipase. In a related aspect of the present invention there is provided a method of treatment or prophylaxis of atherosclerosis by selectively inhibiting ApoCIII activity in a human host, comprising administering an agent which results in the selective inhibition of ApoCIII activity to a human host in need of such treatment or prophylaxis. In this regard, the agent may be an immunogenic composition or vaccine comprising ApoCIII, or fragment thereof, as the immunogen, or alternatively the agent may be a ligand that is capable of blocking the activity of ApoCIII (in that it abrogates the ApoCIII-mediated inhibition of lipoprotein lipase and the binding of ApoB to its receptor). The preferred ligands in this aspect of the present invention are poly- or monoclonal antibodies.

Preferred methods of treating individuals suffering from Atherosclerosis having elevated levels of circulating ApoCIII in their plasma comprise reducing the levels of circulating ApoCIII, by the administration of a vaccine comprising ApoCIII, or fragment thereof, as an immunogen to said individual. Alternatively, in a related aspect of the present invention there is provided a method of treatment or prophylaxis of atherosclerosis by reducing the levels of circulating ApoCIII in the plasma of a patient, by administration of a ligand that is capable of blocking the activity of ApoCIII, in that it abrogates the ApoCIII-mediated inhibition of lipoprotein lipase and the binding of ApoB to its receptor, to said patient. The preferred ligands in this aspect of the present invention are poly or monoclonal antibodies.

Also provided by the present invention is a method of treatment or prophylaxis of atherosclerosis by reducing the number of ApoCIII molecules which are associated with an ApoB molecule *in situ* in the context of a lipoprotein. In a normal individual there is approximately one ApoB present in an LDL particle, the ApoB being associated with between 1-5 ApoCIII molecules. In diseased individuals the number of ApoCIII molecules may increase to up to 25. Accordingly, there is provided by the present invention a method of treatment or prophylaxis of atherosclerosis by reducing the ratio of ApoCIII molecules per ApoB molecules in the LDL in an individual with atherosclerosis from a high disease state level (approximately 20 to 25:1) to a reduced therapeutic level preferably below 15:1, more preferably below 10:1 and more preferably below 5:1, preferably below 3:1, and most preferably approximately 1:1 ApoC:ApoB. Levels of ApoCIII contined within ApoB-containing lipoproteins may be measured by nephelometry or electro-immunodiffusion (normal range is 2 to 3 mg/dL).
Also provided by the present invention is synthetic or recombinantly produced ApoCIII for use in medicine.
The present invention is illustrated by the following examples:

### Example 1, Peptide synthesis

The ApoCIII peptides (1-79, 1-17, 12-35,45-65 and 45-76 (SEQ ID NO.s 1,2,4,6 and 7 respectively)) were synthesised by the solid phase method (Merrifield, 1986) on an automated synthesiser Model ABI 433A (Applied Biosystems Inc.) using Boc/Bzl strategy on a Boc-Ala-PAM resin for total apo CIII and MBHA resin for the others fragments. Each amino acid was coupled twice by dicyclohexylcarbodiimide/hydroxybenzotriazole without capping. Side chain protecting groups were as follows: Arg(Ts), Asp(Ochex), Glu(Ochex), Lys(2-Cl-Z), His(Dnp), Ser(Bzl), Thr(Bzl), Met(O)and Tyr(Br-Z). According to the sequence, the group Dnp on His was removed from the peptide, prior to the cleavage from its support by treatment with 10% β-mercaptoethanol, 5% diisopropylethylamine in DCM for 2 h and in NMP for 2 h. The peptidyl resin was then treated with 50% TFA in DCM for 20 min to remove the amino-terminal Boc. The peptide was cleaved from the resin and simultaneously deprotected according to a low and high HF procedure: the resin (1g) was treated with anhydrous HF (2.5 mL) in the presence of p-cresol (0.75 g), p-thiocresol (0.25 g) and dimethylsulfide (6.5 mL) at 0°C. After 3 h hydrogen fluoride and dimethylsulfide were removed by vacuum evaporation and the residual scavengers and by products were extracted with diethyl ether. The reaction vessel was recharged with p-cresol (0.75 g), p-thiocresol (0.25 g) and 10 ml of anhydrous HF and the mixture was allowed to react at 0°C for 1.5 h. Hydrogen fluoride was removed by evaporation and the residue was triturated with diethyl ether. The residue was filtered off, washed with diethyl ether and extracted with 200 ml of 10% aqueous acetic acid and lyophilised. The crude product was analysed by reversed-phase HPLC on a Vydac C 18 column (4,6 x 250 mm, 5µ, 100 A) using 60 min linear gradient from 0 to 100% Buffer B (Buffer A: 0.05% TFA in H₂O and Buffer B: 0.05% TFA, 60% CH₃CN in H₂O) at flow rate of 0.7 ml/min and detection was performed at 215 nm. Synthetic peptide were purified by RP-HPLC and were characterised and analysed by HPLC, the molecular mass determined by spectrometry.

### Example 2, Antibody production

The synthetic whole ApoCIII synthesised in Example 1 was used to generate polyclonal antibodies in rabbits. The reactivity of this polyclonal anti-whole ApoCIII antiserum against the other shorter synthetic peptides was then assayed, and fractions of antibody specific for each peptide were purified from the antiserum.

*Immunisation* : Peptide ApoCIII (1-79) was emulsified in complete Freund's adjuvant (CFA) and injected intra-dermally using 500µg peptide per injection for the two first injections followed at 15 day intervals with boosters in the same adjuvant but using 250 µg of peptide. The immune response is monitored by taking test bleeds and ELISA system was used to screen for the anti-peptide activity.

*Isolation of the antibodies from serum:* The positive bleeds are pooled and the polyclonal antibodies were isolated by precipitation with 27% sodium sulfate. Peptide specific antibodies were purified from this antibody pool by affinity chromatography. The peptides produced in example 1 were coupled to CH activated sepharose 4B affinity column chromatography (Pharmacia, Uppsala, Sweden) (Axen and al, 1967) and the whole purified antibody pool was passed through these columns. Non retained proteins on the antigen gel were washed off with phosphate-buffered isotonic saline (PBS: Phosphate 50 mmol/L, pH 7.2, NaCl 150 mmol/L). Non specifically bound fractions on the peptide gel were removed with 25 mmol/L PBS. Elution of polyclonal specific IgG was accomplished using 0.2 M glycine, pH 2.8. Purified antibodies are immediately dialysed against 10 mmol/l PBS and concentrated by ultrafiltration using Amicon system (cut-off 100 kD) (Amicon, Beverly, USA), assayed in terms of proteins content (Lowry and al, 1951), then stored as 1ml aliquots (0,5 mg) at -30°C.

### Example 3, Epitope mapping of ApoCIII

*ELISA.* : Microtiter plates (flat-bottom 96-well EIA; Costar, Dutscher) were washed with 0.1 mol/L phosphate-buffered saline (PBS, pH 7,2) before being coated with 100 µl/well of free peptide (5 µg/ml) (1-17, 12-35, 45-65, 45-76 and ApoCIII (1-79)) and incubated overnight at room temperature. The plates were washed four times with buffer and to minimise the non-specific binding to the microtiter wells, the plates were saturated with 250 µL/well of bovine serum albumin at 3% in 0.1 M PBS buffer and incubated for 1 h at 37°C. The plates were washed four times again and incubated for 2 h at 37°C with 100 µL of the purified anti-whole ApoCIII antibody (produced in example 2), diluted in 1% of bovine serum albumin in 0.1 M PBS buffer, then washed three four times with PBS. To assess the immunological reaction, 100 µL of 10 000-fold diluted, anti-rabbit IgG labelled with peroxidase, in 0.1% of BSA in PBS buffer (Sanofi-Diagnostics Pasteur, Mames-La-Coquette, France) were added to each well. After an incubation for 2 h at 37°C, the plates were washed four times with PBS and 100 µL of substrate solution was added. The substrate solution was prepared as follows: 30 mg of o-Phenylenediamine dihydrochloride were dissolved in 20 ml of 0.1 mmol/L phosphate-citrate buffer, pH 5.5 containing 20 µL of 30% hydrogen peroxide. After 30 min at room temperature in the dark, the reaction was stopped by adding to each well 100 µl of 1 mmol/L HCl. The absorbance was measured at 492 nm.

### Results

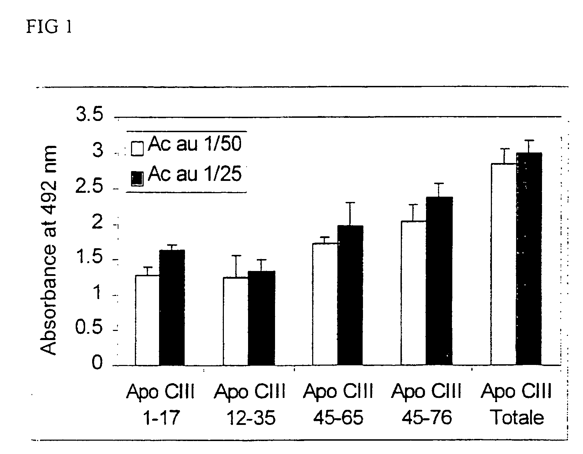

The purified polyclonal antibody generated against the total synthetic apo CIII was used to test its reactivity with the peptides in the ELISA, and also against whole ApoCIII ("ApoCIII totale"). For the results see FIG 1. The results showed that the antibody generated against total ApoCIII recognises all the peptides 1-17, 12-35, 45-65 and 45-76.

### Example 4, Affinity of the antibodies to ApoCIII present in lipoproteins

The objective was to check if the different epitopes corresponding to the peptides are accessible in the lipoproteins and to determine the affinity of each antibody fraction to human plasma-purified lipoproteins (HDL, VLDL) and against whole ApoCIII.

### Materials and methods

The peptide specific antibody fractions were prepared by immunoaffinity to different peptides coupled to CH sepharose as described in Example 2.

*Sandwich ELISA*: Microtiter plates (flat-bottom 96-well EIA; Costar, Dutscher) were washed with 0.1 mol/L phosphate-buffered saline (PBS, pH 7.2) before being coated with 100 µL/well of the affinity purified peptide specific antibodies (10 mg/L) and incubated overnight at room temperature. The plates were then washed four times with buffer and incubated for 2 h at 37°C with 100 µL of dilutions of a sample (4 different samples were sed 1. a standard protein of purified human plasma ApoCIII, 2. human HDL, 3. human VLDL and 4. synthetic ApoCIII (1-79)). To minimise the non-specific binding to the microtiter wells, the dilutions of antigen were performed in 1% of bovine serum albumin in 0.1 M PBS buffer. To assess the immunological reaction, 100 µL of 2 500-fold diluted, polyclonal anti-whole ApoCIII antibody labelled with peroxidase, in 0.1% of BSA in PBS buffer were added to each well. After an incubation for 2 h at 37°C, the plates were washed four times with PBS and 100 µL of substrate solution was added. The substrate solution was prepared as follows: 30 mg of o-Phenylenediamine dihydrochloride (Sigma Chemical Co., St Louis, MO) were dissolved in 20 mL of 0.1 mmol/L phosphate-citrate buffer, pH 5.5 containing 20 µL of 30% hydrogen peroxide. After 30 min at room temperature in the dark, the reaction was stopped by adding to each well 100 µL of 1 mol/L HCl. The absorbance was read at 492 nm.

### Results

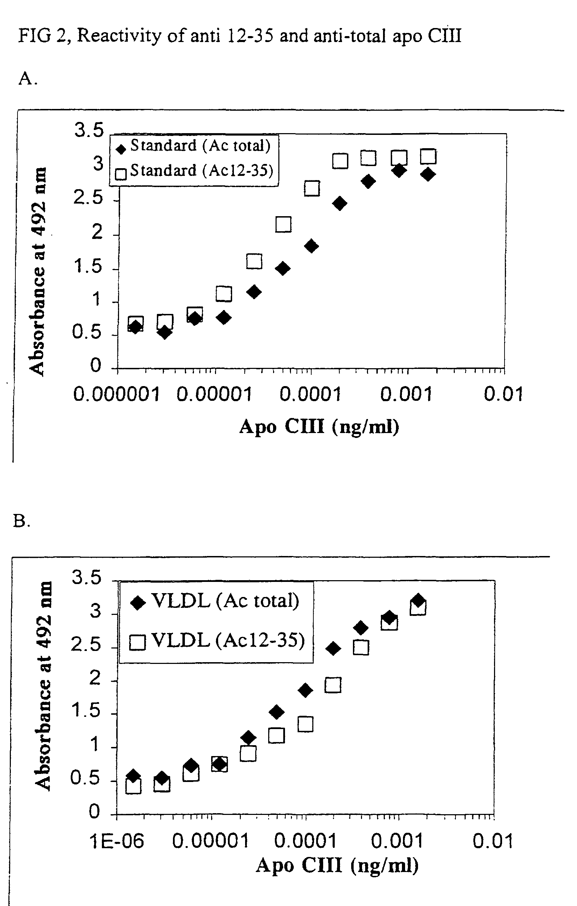

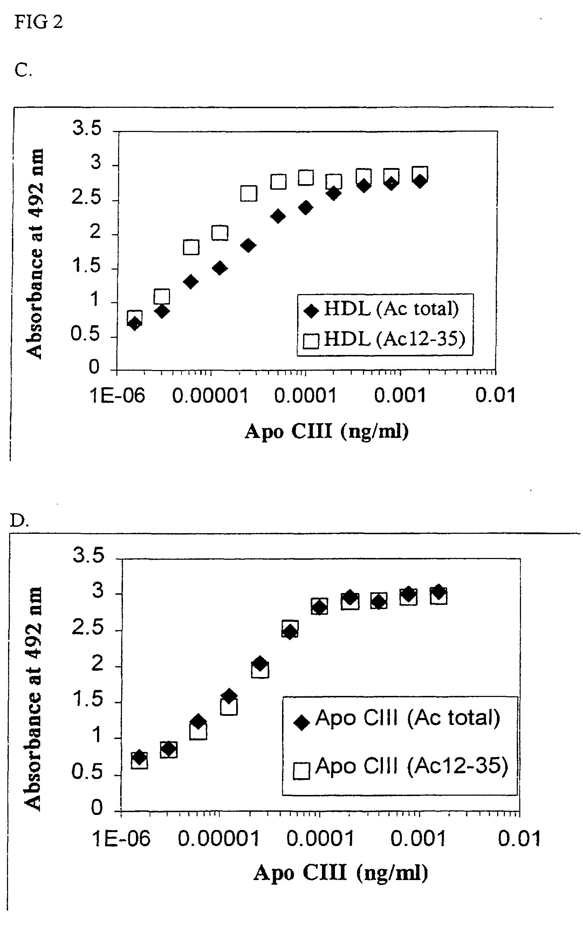

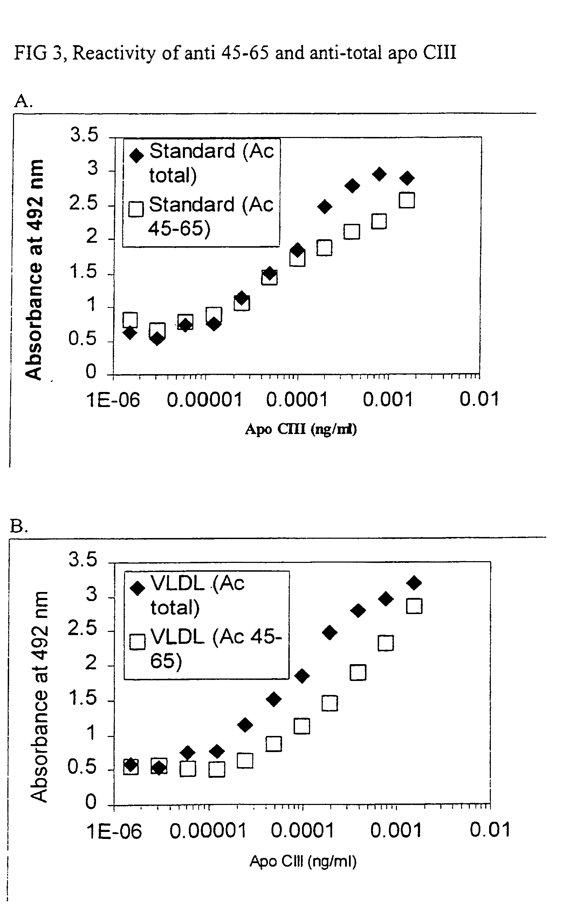

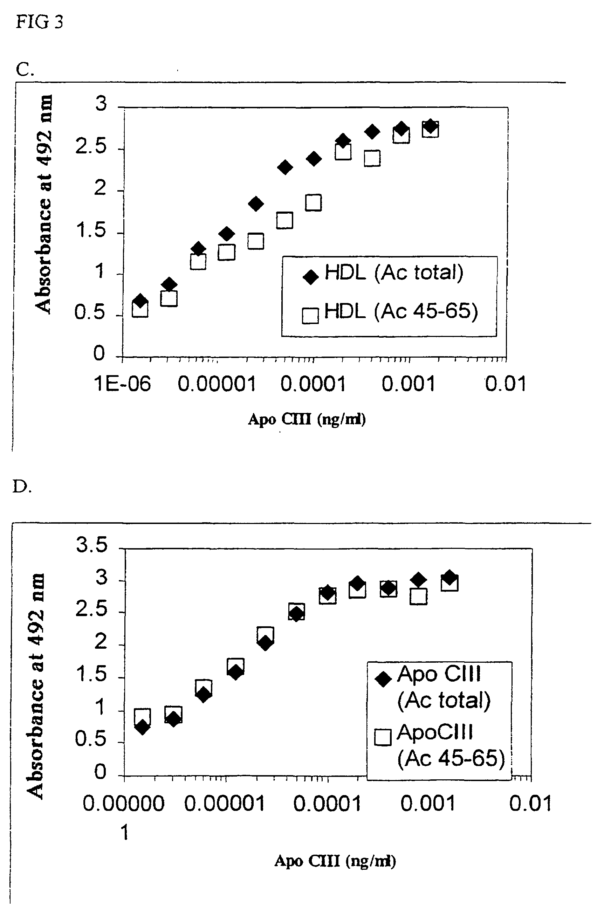

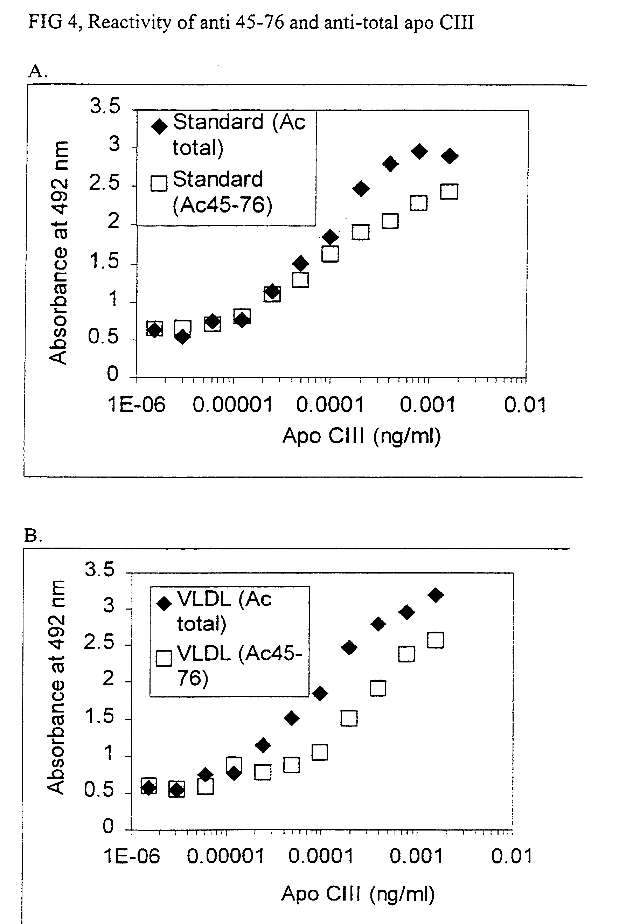

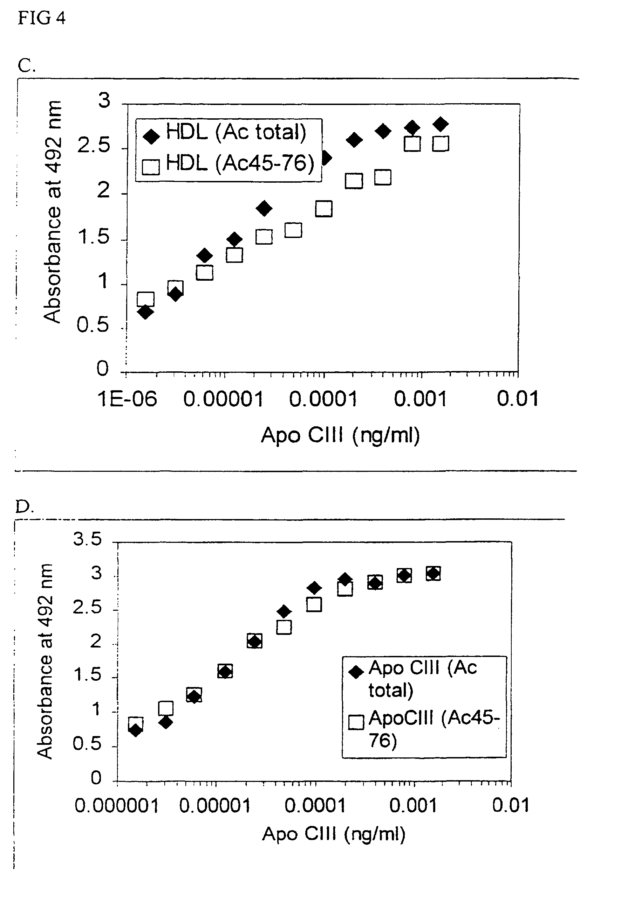

The results show that all the antibodies anti-12-35 (FIG. 2), anti-45-65 (FIG. 3) and anti-45-76 (FIG. 4) recognise the free synthetic ApoCIII in solution (Graph A) with approximately the same affinity as the polyclonal antibody generated against the total ApoCIII ("Ac total" - whole rabbit immunoglobulin). Graph B shows recognition of VLDL, Graph C shows recognition of HDL and graph D shows recognition of ApoCIII (1-79). Of the peptide specific antibodies, anti-12-35 has the highest affinity for ApoCIII in the plasma standard and HDL. Also the reactivity of anti( 12-35) with VLDL similar to that obtained with the whole anti-apo CIII rabbit polyclonal antibody.

### Example 5, Effect of different antibodies on the incorporation of apo CIII into VLDL.

The objective is to determine the capacity of each antibody to inhibit the association of Apo CIII with VLDL. The VLDL fractions were prepared by ultracentrifugation from normotriglyceridemic (NTG) and hypertriglyceridemic (HTG) patients using conventional techniques. A third fraction of VLDL was prepared from the NTG group, where additional ApoCIII was loaded *in vitro* into the VLDL particles (VLDL enriched or VLDL E-CIII).

*Radiolabelling:* The purified Apo CIII was radioiodinated by Bilheimer's modification of McFarlane's method (Bilheimer et a). 1972. *Biochim*. *Biophys*. *Acta,* 260, 212-221 ). Resin AG 2-X8 is regenerated with NaOH 1M (5 minutes), washed with distilled water, then saturated with PBS 0.01 M BSA 1% (w/v). Apo CIII is dialysed against PBS-EDTA 0.01M. 0.5 mCi ¹²⁵I are added to 0.1 ml radiolabeled buffer (glycine 1M, NaCl 1M) containing 5µl 0.033 M ICl solution. The amount of the prepared solution is mixed to 50 µl of the washed resin. Resin with free ¹²⁵I is then removed by centrifugation (1 minute at 62200 rpm), then recovered apo CIII are dialysed against PBS-ADTA 0.01M.

*Incorporation of apo CIII in VLDL (in the presence or not of anti apo CIII)*: 20 µg of each anti-apo CIII antibody is incubated 2 h at 37°C with equivalent amount of ¹²⁵I-apo CIII. Lipoproteins (VLDL NTG, VLDL HTG or VLDL E-CIII) and antibody-¹²⁵I-apo CIII are mixed (w/w); incubated for 1 h at 37 °C, the non-bound ¹²⁵I -apo CIII is dialysed. Measurements of ¹²⁵I radioactivity in ¹²⁵I-apo CIII bound to lipoparticles and in non bound ¹²⁵I-apo CIII is performed to have the rate of the capacity of antibodies to inhibit the association between apo CIII and lipoproteins.

### Results

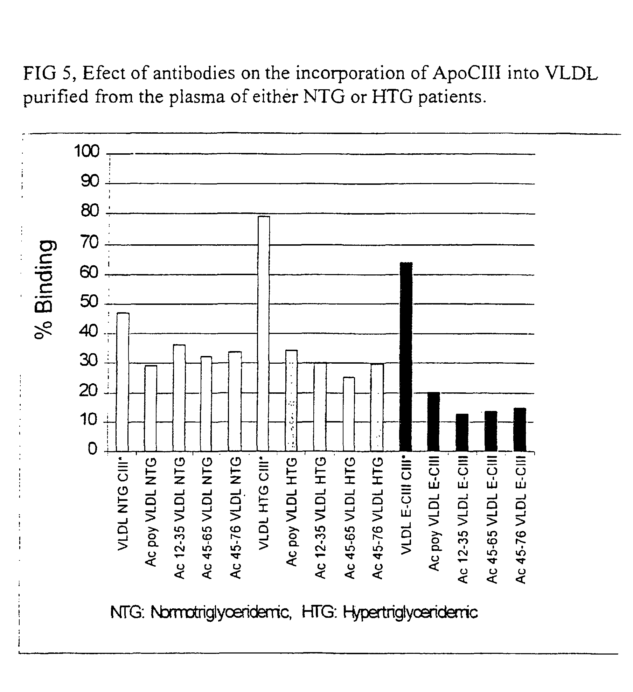

The results in FIG 5, show that all the antibodies (anti-whole ApoCIII or peptide specific) have an effect on the incorporation of apo CIII in the VLDL comparable to the anti-apo CIII polyclonal antibody.

### Example 6, The effect of the ApoCIII specific antibodies on lipoprotein lipase activity

The objective is to test the ability of the antibodies to protect the lipoprotein lipase from the inhibitory effect by the apo CIII.

### Assay of lipolysis with heparan sulfate proteoglycan-bound lipoprotein lipase

The assay was performed in 96-well microtiter plates. Wells were incubated with 0.5 µg of heparan sulfate proteoglycan (HSPG) in 100 µl of PBS 0.1M NaCl 0.15 M pH 7.2-7.4 for 18 h at 4°C, washed 3 times with PBS and subsequently blocked for 1 hour 37°C with PBS containing 1% (w/v) essentially free fatty acid bovine serum albumin (BSA). Then, the wells were incubated with 2 µg of lipoprotein lipase (LPL) in 100 µl of 0.1 M Tris 20% glycerol (v/v), pH 8.5 for 2 h at 4°C. Non fixed LPL was washed 3 times with Tris buffer (0.1M Tris, pH 8.5).
4 fractions of VLDL were then prepared: P1 comprised human plasma-purified VLDL which was enriched in vitro with ApoB; P2 comprised human plasma-purified VLDL which was enriched in vitro with ApoB and ApoE. Other fractions were prepared from P 1 and P2 by enrichment with ApoCIII (P1/ApoCIII enriched and P2/ApoCIII enriched). Some of these 4 fractions were then incubated with the rabbit anti-whole ApoCIII.
Lipolysis was then started by adding of 100 µl of the lipoproteins fractions to the LPL coated plates (0.055 to 0.5 mg/ml of 0.1M Tris, pH 8.4 and 1.5 % BSA (w/v)), and the incubation was performed at 37°C. The reaction was stopped after 20 min by the addition of 100 µl of Tris buffer, Triton X-100 (2% (v/v), final concentration). 100 µl of lipolysed lipoproteins was sampled from each well, cooled at -20°C then the concentration of free fatty acid was measured using NEFA-C kit (Wako chemicals Neuss, Germany) according to the instructions of manufacture.

### Results

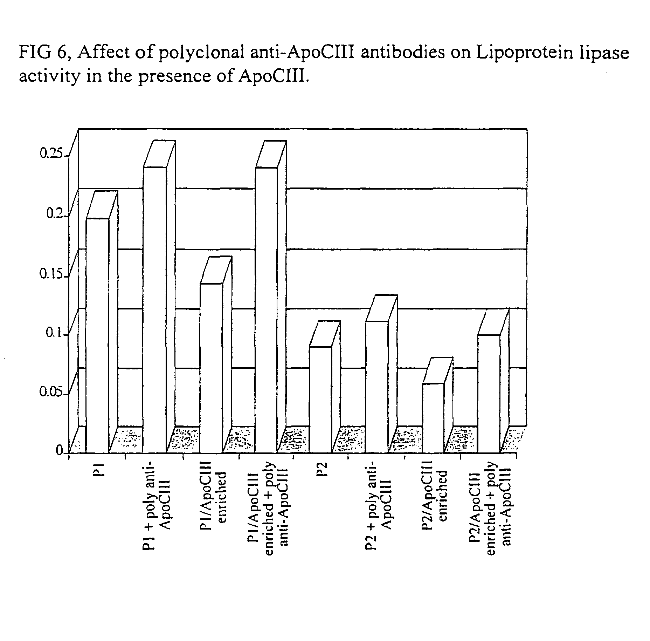

The results are shown in FIG 6., in all cases the activity of the lipoprotein lipase was increased in the presence of the anti-ApoCIII antibodies.

### Example 7, Effect of the antibodies on cholesterol efflux.

The objective is to test for possible negative effects of anti-ApoCIII antibodies on HDL.
*1. Isolation of lipoproteins* : HDL was isolated from fresh normolipemic human plasma at density 1,063-1,21 by sequential ultracentrifugation using Kbr for density adjustments, washed by refutation at d=1,21 and dialyzed against 0,01 M phosphate buffered saline, pH 7.4.
*2. Isolation of Apo-CIII containing lipoproteins by immunaffinity chromatographie :* Apo-CIII containing HDL and Apo-CIII non containing HDL were prepared by immunoaffinity chromatography , using anti-CIII antibodies coupled to activated sepharose 4B. Apo-CIII containing HDLare eluted by using 0,01 M phosphate buffered saline ,pH 7.4, EDTA 0.1 g/L and the Apo-CIII non containing HDL are eluted by using 3M Sodium thiocyanate. Apo CIII containing HDL are dialysed against 0,01 M phosphate buffered saline, pH 7.4, EDTA 0.1 g/L. The pure fraction of HDL was called HDLt, the retained fraction of ApoCIII containing HDL was called HDL CIII, and the non-adsorbed HDL fraction was called "HDL non CIII".
*3. Seeding conditions* : Fu5AH which arc rat hepatica cells were seeded in 12-well plates at 25000 cells/mL and 2 mL in each well, and cultured in minimal essential medium 95% (GIBCOBRL) supplemented with Penicillin (100 µg/mL), Streptomycin (100 g/mL), Glutamin (2mM) and 5% (vol/vol) new born calf serum. Cells were grown for 2 days at 37°C in a humidified 5% CO₂ atmosphere before the cellular lipid radiolabelling.
   We used 3 wells per sample and to control the efflux validity we used :
   - a negative control : 1 mL of minimal essential medium
   - a positive control : HDL₃ at 100 µg/mL
   - an internal control : human normolipemic plasma pool stored at -20°C and used at 2,5% (vol/vol)
   - a control of labelled cholesterol loading : The radioactivity of cells was measured before incubation with the samples.
*4. Radiolabeling of Fu5AH cells* : - Radiolabelled cholesterol [1α,2α,(n)-³H]cholesterol was added to the cells to have a final concentration of 1 µCi/well :
   - Evaporate *X* µCi under N₂
   - Add 1 mL of ethanol and incubate 45 to 60 minutes at 60°C.
   - Evaporate under N₂
   - Add 50 µL of ethanol and incubate 15 to 30 minutes at 60 °C.
   - Add an equal volume of minimal essential medium (MEM) and of new born calf serum to have a final concentration of 5% in MEM and incubate 30 minutes at 37°C.
   - Add MEM to have the final volume.
   - Cells were grown during 3 days in the presence of radiolabel (2 mL/well)
*5. Cholesterol efflux* : To ensure that the label was evenly distributed among cellular pools, the labelling medium was replaced with MEM containing 0,5% bovine serum albumin (BSA) 24 hours before the efflux.
   - Washed one time with cellular phosphate-buffered saline (PBS) and incubate during 3 hours at 37°C, 5% CO₂ the HDL to a final concentration of 50 µg/mL (500 µL/well). Before the incubation with cells, HDL were preincubated with the different antibodies at 15 µg/mL during 2 hours at 37°C.
   - The efflux phase was ended by removing the serum-containing medium from each well and then we washed 3 times the cells with PBS Cells were remove in 500 µL of sodium hydroxyde (0,1 mol/L) per well.
   - The counting is realised on 250 µL of medium or cellular suspension which we added 4 mL of a scintillation cocktail (Optiphase 'Hisafe' 3, WALLAC) ; with a liquid scintillation counter (WALLAC 1410) during one minute.
   The efflux percentage is calculated like shown :
   **[dpm medium / (dpm medium+dpm cells)]*100**

### Results

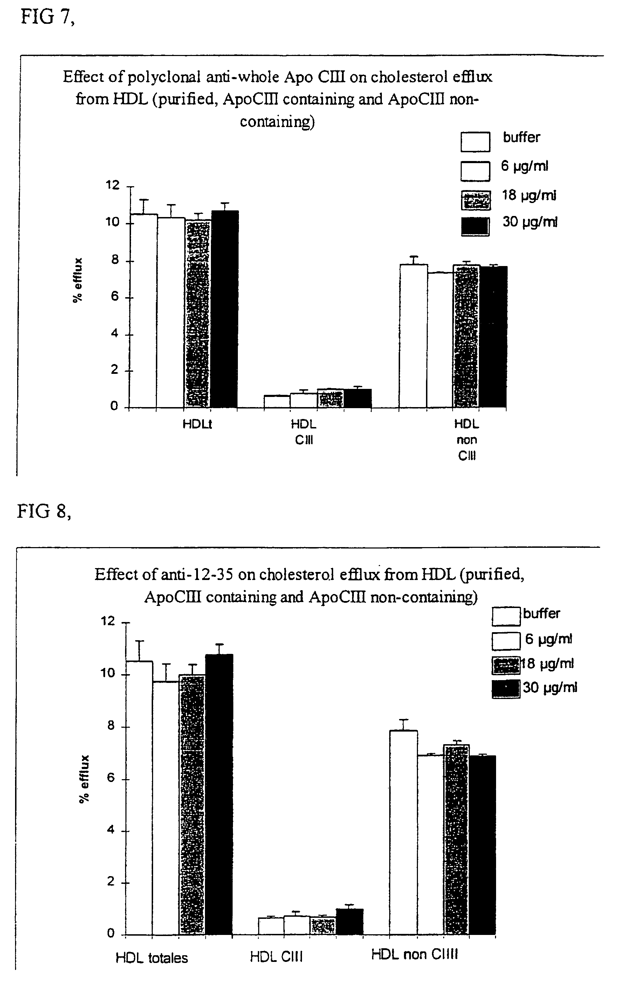

The ApoCIII containing HDL were separated from the non-ApoCIII containing HDL by immunoaffinity. The cholesterol efflux was measured in different fractions (total HDL, HDL-CIII and HDH non CIII) with and without the anti-whole ApoCIII or anti 12-35 antibody at different concentrations. The results are show in FIGs 7 and 8. It was confirmed with all the experiments that the antibodies do not affect HDL function in terms of cholesterol efflux.

### Example 8, Manufacture and immunogenicity of peptide conjugates

Immunogens comprising peptides 12-35 and 45-65 conjugated to bovine serum albumin (BSA) were prepared, formulated into vaccines, and administered intramuscularly in mice.

Four peptides were synthesised, a pair of peptides based on the sequence 12-35 and another pair based on the sequence 45-65 of ApoCIII. Each pair of peptides had a GGC linker incorporated onto either the N- or C- terminus of the peptide:

The peptides were conjugated to a BSA as a protein carrier by Maleimide chemistry. BSA was purchased from Pierce, which was pre-activated with a succinimidyl 4-(N-maleimidomethyl)-cyclohexane-1-carboxylate (SMCC) linker. SMCC may also be bought from any major manufacturer and used following the manufacturers instructions. The coupling of the BSA to the carrier via the SMCC was carried out over 2 hr at room temperature with an excess of peptide, before quenching with the reaction with excess cystein, followed by dialysis against phosphate buffer.

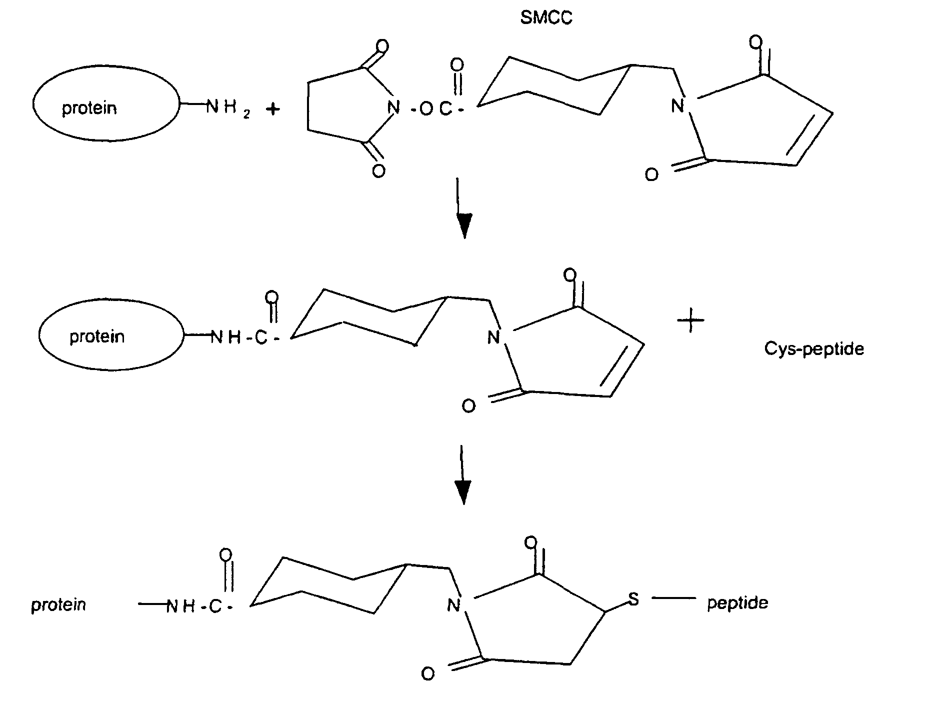

Analysis of the resultant soluble conjugate immunogens showed that there were around 19 peptides conjugated onto each BSA carrier molecule.

The immunogens were all formulated into vaccines by admixture with an adjuvant system comprising the saponin QS21, 3-de-O-acylated monophosphoryl lipid A (3D-MPL) and an oil in water emulsion (with squalene and α-tocopherol oil phase) as described in WO 95/17210. The vaccines were then administered to groups of 10 BalbC mice, containing 25 µg of immunogen, intramuscularly on days 0, 14 and 28; and serum samples were taken on day 28 and day 42. The sera were then analysed for anti-whole ApoCIII titres and anti-peptide titres by ELISA assay (where the plates were coated with either whole ApoCIII or corresponding peptide), and the results expressed as Mid point titres.

### Results

The results showed that the peptide immunogens produced were highly immunogenic, and induced antibodies that cross reacted strongly with native whole ApoCIII. The results obtained in the groups were highly homogenous, and showed a strong boost after the third admistration. The results for each mouse in the groups of 10 are shown in table 1.

**Table 1.**

| Murine anti-peptide IgG titres responses post II (day 28). | | | | |
|---|---|---|---|---|
| | Anti- peptide IgG responses (post II) | | | |
| Mouse No. | 12-35_{COOH}-BSA | 12-35_{NH2}-BSA | 45-65_{COOH}-BSA | 45-65_{NH2}-BSA |
| 1 | 8930 | 7147 | 3481 | 6091 |
| 2 | 74950 | 10459 | 1128 | 12112 |
| 3 | 24889 | 8825 | 1847 | 3780 |
| 4 | 51084 | 11922 | 1907 | 9289 |
| 5 | 34756 | 9084 | 2217 | 6563 |
| 6 | 19427 | 3346 | 1068 | 4283 |
| 7 | 66607 | 1677 | 1794 | 773 |
| 8 | 13579 | 7020 | 907 | 3269 |
| 9 | 14493 | 15311 | 2434 | 939 |
| 10 | 30499 | 43065 | 1318 | 12667 |
| Average | **33921** | **11786** | **1810** | **5977** |
| Geomean | **27297** | **8450** | **1672** | **4305** |
| Standard Deviation | **23013** | **11673** | **777** | **4232** |

**Table 2.**

| Murine anti-peptide IgG titres post III (day 42). | | | | |
|---|---|---|---|---|
| | Anti- peptide IgG responses (post III) | | | |
| Mouse No. | 12-35_{COOH}-BSA | 12-35_{NH2}-BSA | 45-65_{COOH}-BSA | 45-65_{NH2}-BSA |
| 1 | 69735 | 11369 | 9667 | 3005 |
| 2 | 123458 | 17581 | 3440 | 15804 |
| 3 | 68283 | 7867 | 17535 | 5826 |
| 4 | 75884 | 22679 | 20866 | 23188 |
| 5 | 34135 | 12938 | 16917 | 11930 |
| 6 | 82473 | 11664 | 7353 | 8013 |
| 7 | 163248 | 7996 | 3620 | 172 |
| 8 | 51196 | 10605 | 7859 | 2464 |
| 9 | 65127 | 11165 | 6122 | 2026 |
| 10 | 59430 | 25079 | 6304 | 23379 |
| Average | **79297** | **13894** | **9968** | **9581** |
| Geomean | **72590** | **12902** | **8357** | **5117** |
| Standard Deviation | **37501** | **5940** | **6208** | **8666** |

**Table 3,**

| Murine anti-whole ApoCIII IgG titres post II (day 28). | | | | |
|---|---|---|---|---|
| | Anti- whole ApoCIII IgG responses (post II) | | | |
| Mouse No. | 12-35_{COOH}-BSA | 12-35_{NH2}-BSA | 45-65_{COOH}-BSA | 45-65_{NH2}-BSA |
| 1 | 607 | 2618 | 9531 | 786 |
| 2 | 4471 | 2176 | 7997 | 3854 |
| 3 | 2193 | 250 | 763 | 2370 |
| 4 | 6118 | 649 | 4728 | 475 |
| 5 | 3587 | 775 | 6890 | 3816 |
| 6 | 3012 | 1223 | 9924 | 3627 |
| 7 | 3350 | 250 | 8340 | 250 |
| 8 | 1853 | 718 | 6079 | 250 |
| 9 | 1155 | 4791 | 10037 | 2618 |
| 10 | 1056 | 2730 | 1948 | 6067 |
| Average | **2740** | **1618** | **6624** | **2411** |
| Geomean | **1718** | **1452** | **3266** | **1963** |
| Standard Deviation | **2225** | **1075** | **5334** | **1469** |

**Table 4,**

| Murine anti-whole ApoCIII IgG titres post III (day 42). | | | | |
|---|---|---|---|---|
| | Anti- whole ApoCIII IgG responses (post III) | | | |
| Mouse No. | 12-35_{COOH}-BSA | 12-35_{NH2}-BSA | 45-65_{COOH}-BSA | 45-65_{NH2}-BSA |
| 1 | 14231 | 16618 | 18010 | 1182 |
| 2 | 46994 | 26128 | 10152 | 6866 |
| 3 | 19189 | 5342 | 2392 | 5460 |
| 4 | 32417 | 18256 | 12061 | 635 |
| 5 | 7506 | 10740 | 17636 | 7448 |
| 6 | 35724 | 9794 | 13494 | 4490 |
| 7 | 24471 | 5681 | 9274 | 50 |
| 8 | 13474 | 4658 | 11230 | 606 |
| 9 | 25203 | 23127 | 12450 | 9445 |
| 10 | 5196 | 24516 | 1744 | 24066 |
| Average | **22441** | **14486** | **10844** | **6025** |
| Geomean | **13205** | **8322** | **5436** | **7150** |
| Standard Deviation | **18547** | **12092** | **8817** | **2405** |

## Claims

1. An immunogenic composition suitable for administration to a human host for the treatment or prevention of atherosclerosis, having an immunogen comprising an apolipoprotein or peptide or fragment thereof, which in its full length native form the apolipoprotein has at least one of the following activities (a) the inhibition of the binding of Apolipoprotein B to its receptor, and/or (b) the inhibition of lipoprotein lipase, and wherein the immunogenic composition is capable of inducing an immune response in a human host.

2. The immunogenic composition as claimed in claim 1, wherein the full length native form of the apalipoprotein has both of the activities.

3. The immunogenic composition as claimed in claim 1, wherein the apolipoprotein is ApoCIII or fragment, peptide, or mimotope thereof.

4. The immunogenic composition as claimed in claim 3 wherein the ApoCIII or fragment, peptide, or mimotope thereof is conjugated or fused to a protein carrier.

5. The immunogenic composition as claimed in any one of claims 1 to 4, wherein the immunogen is any one of the sequences selected from SEQ ID NO.1-7.

6. A vaccine comprising the immunogenic composition as claimed in any one of claims 1 to 5, and an adjuvant.

7. The use of an agent which results in the inhibition of at least one of the following activities of an atherogenic apolipoprotein in a human host in need of such treatment or prophylaxis, (a) the inhibition of the binding of Apolipoprotein B to its receptor, and/or (b) the inhibition of lipoprotein lipase is the preparation of a medicament for treatment or prophylaxis of atherosclerosis by selectively inhibiting the activity of an atherogenic apolipoprotein in a human host.

8. The use of an agent which results in the selective inhibition of ApoGIII activity in a human host in need of such treatment or prophylaxis in the preparation of a medicament for the treatment or prophylaxis of atherosclerosis by selectively inhibiting ApoCIII activity in a human host.

9. A use claimed in claim 7 or 8, therein the agent is a vaccine comprising ApoCITI, or fragment thereof, as an immunogen.

10. A use as claimed in claim 7 or 8, wherein the agent is a ligand of ApoCIII.

11. A use as claimed in claim 10, wherein the ligand is an antibody.

12. A use as claimed in claims 7 to 11, wherein the agent blocks the ApoCIII-mediated inhibition of lipoprotein lipase and the binding of ApoB to its receptor.

## Patentansprüche

1. Immunogene Zusammensetzung, die zur Verabreichung an einen menschlichen Wirt zur Behandlung oder Prävention von Atherosklerose geeignet ist, mit einem Immunogen, das ein Apolipoprotein oder ein Peptid oder Fragment davon umfasst, worin das Apolipoprotein in seiner nativen Form voller Länge wenigstens eine der folgenden Aktivitäten aufweist: (a) die Inhibierung der Bindung von Apolipoprotein B an seinen Rezeptor und/oder (b) die Inhibierung von Lipoproteinlipase, und worin die immunogene Zusammensetzung eine Immunreaktion in einem menschlichen Wirt induzieren kann.

2. Immunogene Zusammensetzung gemäss Anspruch 1, worin die native Form voller Länge des Apolipoproteins beide Aktivitäten aufweist.

3. Immunogene Zusammensetzung gemäss Anspruch 1, worin das Apolipoprotein ApoCIII oder ein Fragment, Peptid oder Mimotop davon ist.

4. Immunogene Zusammensetzung gemäss Anspruch 3, worin das ApoCIII oder Fragment, Peptid oder Mimotop davon an einen Proteinträger konjugiert oder fusioniert ist.

5. Immunogene Zusammensetzung gemäss einem der Ansprüche 1 bis 4, worin das Immunogen eine der aus SEQ ID NO: 1-7 ausgewählten Sequenzen ist.

6. Impfstoff, der die immunogene Zusammensetzung gemäss einem der Ansprüche 1 bis 5 und einen Hilfsstoff umfasst.

7. Verwendung eines Mittels, das zur Inhibierung wenigstens einer der folgenden Aktivitäten eines atherogenen Apolipoproteins in einem menschlichen Wirt, der einer solchen Behandlung oder Prophylaxe bedarf, führt: (a) die Inhibierung der Bindung von Apolipoprotein B an seinen Rezeptor und/oder (b) die Inhibierung von Lipoproteinlipase, in der Herstellung eines Medikaments zur Behandlung oder Prophylaxe von Atherosklerose durch selektive Inhibierung der Aktivität eines atherogenen Apolipoproteins in einem menschlichen Wirt.

8. Verwendung eines Mittels, das zur selektiven Inhibierung von ApoCIII-Aktivität in einem menschlichen Wirt, der einer solchen Behandlung oder Prophylaxe bedarf, führt, in der Herstellung eines Medikaments zur Behandlung oder Prophylaxe von Atherosklerose durch selektive Inhibierung von ApoCIII-Aktivität in einem menschlichen Wirt.

9. Verwendung gemäss Anspruch 7 oder 8, worin das Mittel ein Impfstoff ist, der ApoCIII oder ein Fragment davon als Immunogen umfasst.

10. Verwendung gemäss Anspruch 7 oder 8, worin das Mittel ein Ligand von ApoCIII ist.

11. Verwendung gemäss Anspruch 10, worin der Ligand ein Antikörper ist.

12. Verwendung gemäss Ansprüchen 7 bis 11, worin das Mittel die ApoCIII-vermittelte Inhibierung von Lipoproteinlipase und die Bindung von ApoB an seinen Rezeptor blockiert.

## Revendications

1. Composition immunogène adaptée pour administration à un hôte humain pour le traitement ou la prévention de l'athérosclérose ayant un immunogène comprenant une apolipoprotéine ou un peptide ou un fragment de celle-ci dans laquelle, sous sa forme native entière, l'apolipoprotéine a au moins une des activités suivantes (a) l'inhibition de la liaison de l'Apolipoprotéine B à son récepteur et/ou (b) l'inhibition de la lipoprotéine lipase et dans laquelle la composition immunogène est capable d'induire une réponse immunitaire chez un hôte humain.

2. Composition immunogène selon la revendication 1, dans laquelle la forme native entière de l'apolipoprotéine a ces deux activités.

3. Composition immunogène selon la revendication 1, dans laquelle l'apolipoprotéine est l'ApoCIII ou un fragment, un peptide ou un mimotope de celle-ci.

4. Composition immunogène selon la revendication 3 dans laquelle l'ApoCIII ou un fragment, un peptide ou un mimotope de celle-ci est conjugué ou fusionné avec un véhicule protéique.

5. Composition immunogène selon l'une quelconque des revendications 1 à 4, dans laquelle l'immunogène est l'une quelconque des séquences choisies parmi SEQ ID No. 1 à 7.

6. Vaccin comprenant la composition immunogène selon l'une quelconque des revendications 1 à 5 et un adjuvant.

7. Utilisation d'un agent qui conduit à l'inhibition d'au moins une des activités suivantes d'une apolipoprotéine athérogène chez un hôte humain nécessitant un tel traitement ou prophylaxie, (a) l'inhibition de la liaison de l'Apolipoprotéine B à son récepteur et/ou (b) l'inhibition de la lipoprotéine lipase, dans la préparation d'un médicament pour le traitement ou la prophylaxie de l'athérosclérose en inhibant sélectivement l'activité d'une apolipoprotéine athérogène chez un hôte humain.

8. Utilisation d'un agent qui provoque l'inhibition sélective de l'activité de l'ApoCIII chez un hôte humain nécessitant un tel traitement ou prophylaxie dans la préparation d'un médicament pour le traitement ou la prophylaxie de l'athérosclérose en inhibant sélectivement l'activité de l'ApoCIII chez un hôte humain.

9. Utilisation selon la revendication 7 ou 8, dans laquelle l'agent est un vaccin comprenant l'ApoCIII, ou un fragment de celle-ci, en tant qu'immunogène.

10. Utilisation selon la revendication 7 ou 8, dans laquelle l'agent est un ligand de l'ApoCIII.

11. Utilisation selon la revendication 10, dans laquelle le ligand est un anticorps.

12. Utilisation selon les revendications 7 à 11, dans laquelle l'agent bloque l'inhibition à médiation par l'ApoCIII de la lipoprotéine lipase et la liaison de l'ApoB à son récepteur.


### SEQUENCE LISTING

<110> SmithKline Beecham Biologicals S.A.
<120> Vqccine
<130> B45212
<160> 11
<170> FastSEQ for Windows Version 3.0
<210> 1
<211> 79
<212> PRT
<213> Human ApoCIII peptides
<400>
   Ser Glu Ala Glu Asp Ala Ser Leu Leu Ser Phe Met Gln Gly Tyr Met
    1               5                  10                  15
   Lys His Ala Thr Lys Thr Ala Lys Asp Ala Leu Ser Ser Val Gln Glu
               20                  25                  30
   Ser Gln Val Ala Gln Gln Ala Arg Gly Trp Val Thr Asp Gly Phe Ser
           35                  40                  45
   Ser Leu Lys Asp Tyr Trp Ser Thr Val Lys Asp Lys Phe Ser Glu Phe
       50                  55                  60
   Trp Asp Leu Asp Pro Glu Val Arg Pro Thr Ser Ala Val Ala Ala
   65                  70                  75
<210> 2
<211> 17
<212> PRT
<213> Human ApoCIII peptides
<400> 2
   Ser Glu Ala Glu Asp Ala Ser Leu Leu Ser Phe Met Gln Gly Tyr Met
    1               5                  10                  15
   Lys
<210> 3
<211> 40
<212> PRT
<213> Human ApoCIII peptides
<400> 3
   Ser Glu Ala Glu Asp Ala Ser Leu Leu Ser Phe Met Gln Gly Tyr Met
    1              5                  10                  15
   Lys His Ala Thr Lys Thr Ala Lys Asp Ala Leu Ser Ser Val Gln Glu
               20                  25                  30
   Ser Gln Val Ala Gln Gln Ala Arg
           35                  40
<210> 4
<211> 24
<212> PRT
<213> Human ApoCIII peptides
<400> 4
   Met Gln Gly Tyr Met Lys His Ala Thr Lys Thr Ala Lys Asp Ala Leu
    1              5                  10                  15
   Ser Ser Val Gln Glu Ser Gln Val
               20
<210> 5
<211> 39
<212> PRT
<213> Human ApoCIII peptides
<400> 5
   Gly Trp Val Thr Asp Gly Phe Ser Ser Leu Lys Asp Tyr Trp Ser Thr
    1              5                  10                  15
   Val Lys Asp Lys Phe Ser Glu Phe Trp Asp Leu Asp Pro Glu Val Arg
               20                  25                  30
   Pro Thr Ser Ala Val Ala Ala
           35
<210> 6
<211> 21
<212> PRT
<213> Human ApoCIII peptides
<400> 6
   Asp Gly Phe Ser Ser Leu Lys Asp Tyr Trp Ser Thr Val Lys Asp Lys
    1              5                  10                  15
   Phe Ser Glu Phe Trp
               20
<210> 7
<211> 32
<212> PRT
<213> Human ApoCIII peptides
<400> 7
   Asp Gly Phe Ser Ser Leu Lys Asp Tyr Trp Ser Thr Val Lys Asp Lys
    1              5                  10                  15
   Phe Ser Glu Phe Trp Asp Leu Asp Pro Glu Val Arg Pro Thr Ser Ala
               20                  25                  30
<210> 8
<211> 27
<212> PRT
<213> Human ApoCIII peptides
<400> 8
   Met Gln Gly Tyr Met Lys His Ala Thr Lys Thr Ala Lys Asp Ala Leu
    1              5                   10                  15
   Ser Ser Val Gln Glu Ser Gln Val Gly Gly Cys
               20                  25
<210> 9
<211> 27
<212> PRT
<213> Human ApoCIII peptides
<400> 9
   Cys Gly Gly Met Gln Gly Tyr Met Lys His Ala Thr Lys Thr Ala Lys
    1              5                   10                  15
   Asp Ala Leu Ser Ser Val Gln Glu Ser Gln Val
               20                  25
<210> 10
<211> 24
<212> PRT
<213> Human ApoCIII peptides
<400> 10
   Asp Gly Phe Ser Ser Leu Lys Asp Tyr Trp Ser Thr Val Lys Asp Lys
    1              5                   10                  15
   Phe Ser Glu Phe Trp Gly Gly Cys
               20
<210> 11
<211> 24
<212> PRT
<213> Human ApoCIII peptides
<400> 11
   Cys Gly Gly Asp Gly Phe Ser Ser Leu Lys Asp Tyr Trp Ser Thr Val
    1              5                   10                  15
   Lys Asp Lys Phe Ser Glu Phe Trp
               20